# FASCICULE DE BREVET EUROPEEN

(11) **EP 2 364 659 B1**
(45) Date de publication et mention de la délivrance du brevet: **10.10.2012**
(21) Numéro de dépôt: 11001208.5
(22) Date de dépôt: 15.02.2011
(51) Int. Cl.: A61B 17/80

(54) **Systeme d'osteosynthese a plaque**
Osteosynthesesystem mit Hilfe von Knochenplatten
Osteosynthesis system using bone plates

(30) Priorité: 08.03.2010 FR 1000936
(43) Date de publication de la demande: 14.09.2011
(73) Titulaire: Memometal Technologies, 35170 Bruz (FR)
(72) Inventeur: Martinache, Xavier, 51100 Reims (FR); Papaloizos, Michael, 1204 Geneve (CH); Tchurukdichian, Alain, 21000 Dijon (FR); Prandi, Bernard, 35700 Rennes (FR); Chick, Grégoire, 1227 Carouge-Geneve (CH); Cognet, Jean-Michel, 51100 Reims (FR)
(74) Mandataire: Benech, Frédéric

(56) Documents cités:
- WO-A1-2008/113191
- US-A1- 2009 234 359

## Description

La présente invention concerne un système d'ostéosynthèse pour réduction d'une fracture distale d'os long ou allongé comme le radius, comprenant une plaque du type comportant une partie épiphysaire élargie en forme de palette solidaire d'une partie diaphysaire allongée, lesdites parties étant munies de trous de fixation pour vis d'ancrage, la plaque comportant un orifice oblong pour réglage de la position longitudinale de la plaque sur l'os.

Rappelons que, pour un os, la partie épiphysaire ou épiphyse est la partie distale de l'os et la partie diaphysaire ou diaphyse, la partie proximale du côté du corps de l'os.

L'invention trouve une application particulièrement importante, bien que non exclusive dans le domaine des fractures distales du radius-Mais un tel système peut également être utilisé pour tout type de fracture sur une partie distale d'os long.

Le traitement d'une fracture de l'extrémité d'un os comme le radius présente des difficultés lorsque l'on veut réaliser l'ostéosynthèse de fragments d'os distaux et ce notamment compte tenu de la présence de tendons et de ligaments.

Afin de réduire ces fractures on utilise de façon connue des plaques rigides du type ci-dessus.

On connaît ainsi (US 2009/234359 et WO 2008/113191) des plaques d'ostéosynthèse permettant un positionnement des deux parties osseuses de la fracture avant fixation de la plaque.

De telles plaques présentent cependant des inconvénients.

En effet, elles ne permettent qu'un positionnement en longueur avec ajustage longitudinal grâce au trou oblong, mais sans possibilité d'ajustement transversal.

Concrètement le chirurgien fixe la plaque sur le fragment distal, pose une vis dans le trou oblong et ramène le fragment distal en contact avec la partie proximale de l'os, en faisant glisser la vis dans le trou, jusqu'à ce que les deux parties viennent en butée l'une sur l'autre.

La position adéquate de la plaque est ainsi réalisée sur les os avant de verrouiller ladite plaque dans la diaphyse.

Le chirurgien ne peut donc pas corriger de défaut de positionnement latéral dans le sens transversal, sauf à repercer dans le fragment proximal à côté du premier trou, pour se décaler. Dans la pratique cela s'avère souvent impossible car les deux trous seraient trop proches l'un de l'autre. Il y a alors un dépassement de la partie diaphysaire de la plaque côté proximal, ce qui va en général occasionner une gêne pour le patient.

La présente invention vise à pallier ces inconvénients, en proposant un système d'ostéosynthèse pour réduction de fracture distale répondant mieux que ceux antérieurement connus aux exigences de la pratique, notamment en ce que elle permet la réduction de la fracture en autorisant un positionnement exact en latéral de la partie diaphysaire.

Dans ce but l'invention propose essentiellement un système d'ostéosynthèse pour réduction d'une fracture distale d'un os long ou allongé, comprenant une plaque comportant une partie épiphysaire élargie en forme de palette solidaire d'une partie diaphysaire allongée, lesdites parties étant munies de trous de fixation pour vis d'ancrage, ladite partie diaphysaire comprenant un orifice oblong pour réglage de la position longitudinale de la plaque sur l'os,
caractérisé en ce que la partie épiphysaire présente une base agrandie comprenant un évidement sensiblement triangulaire ou trapézoïdal,
et en ce que le système comporte de plus une pièce de réglage amovible de la plaque sur l'os comprenant une première partie ou partie proximale plus large que le trou oblong munie d'un pion agencé pour être inséré dans ledit trou oblong avec lequel il coopère à glissement, et une deuxième partie ou partie distale de longueur supérieure à la longueur latérale de l'évidement, dont les extrémités sont propres à être en contact avec les bords de l'évidement de la partie épiphysaire lorsque le pion est inséré dans le trou oblong, ladite deuxième partie étant munie d'une lumière allongée en vis à vis de l'évidement, ladite lumière étant propre à la mise en place d'une vis de positionnement, de sorte qu'en déplaçant la vis dans la lumière de la pièce de réglage et/ou le pion le long du trou oblong on déplace longitudinalement et/ou transversalement la plaque.

Une fois la plaque correctement positionnée, le chirurgien peut alors mettre en place les vis d'ancrage par exemple grâce à un bloc guide de vissage, sans risquer de bouger, puis retirer la pièce de réglage amovible.

Ainsi, bien qu'en compliquant le système par ajout d'une pièce amovible, un positionnement impeccable est obtenu.

Dans des modes de réalisation particuliers de l'invention, on a de plus recours à l'une et/ou à l'autre des dispositions suivantes :
- la pièce de réglage amovible est en forme de T, la première partie formant la jambe du T et la deuxième partie la barre du T ;
- la partie diaphysaire est sensiblement allongée dans un premier plan et la partie épiphysaire est sensiblement située dans un deuxième plan incliné par rapport au plan de ladite partie diaphysaire, par exemple de quelques degrés, par exemple 5° à 15°, avantageusement entre 8° et 10°.

De ce fait la pièce en T présente elle aussi un léger coude et le pion une hauteur suffisante, pour conserver une possibilité de guidage lors de l'ajustage longitudinal ;
- le trou oblong est de longueur comprise entre 6 mm et 12 mm, avantageusement de l'ordre de 10 mm ;
- la lumière est de longueur comprise entre 12 mm et 14 mm.

Avantageusement la largeur de la lumière est de l'ordre de 5 mm en proximal et de l'ordre de 12 mm ou plus en distal ;
- l'évidement sensiblement triangulaire présente une surface comprise entre la moitié et les 4/5eme de la base agrandie de la partie épiphysaire.

Le système comporte de plus un bloc guide de vissage épiphysaire comprenant des orifices de guidage propres à être disposés en vis à vis des trous de fixation des vis d'ancrage de la partie épiphysaire.

L'invention propose également un procédé, non revendiqué, de mise en place d'une plaque de réduction du radius telle que décrite ci-avant.

L'invention propose aussi un procédé, non revendiqué, de fixation d'une plaque d'ostéosynthèse sur un os fracturé munie de moyens de déplacement longitudinal, caractérisé en ce que, la plaque étant de plus munie de moyens de déplacement transversal de la plaque par rapport à l'os comprenant une pièce en T ou glisseur, on insère une ou deux vis épiphysaires dans la plaque en vis à vis de l'épiphyse de l'os, on contrôle la position latérale de la plaque, et si la position de la plaque n'est pas bonne, on ajuste la position latérale autour d'une vis du glisseur, puis une fois la position de la plaque recherchée obtenue, on enlève le glisseur et on met en place les dernières vis de fixation.

La présente invention sera mieux comprise à la lecture de la description qui suit d'un mode de réalisation donné à titre d'exemple non limitatif.

La description se réfère aux figures qui l'accompagnent dans lesquelles :
La figure 1 est une vue de dessus d'une plaque selon un mode de réalisation de l'invention, en position sur une extrémité de radius.
La figure 2 montre la même plaque et la pièce de réglage amovible appartenant au système selon le mode de réalisation de l'invention plus particulièrement décrit ici.
La figure 3 montre le système comportant de plus un bloc guide de vissage épiphysaire.
Les figures 4A à 4C montrent le système de la figure 2 dans trois positions différentes, illustrant la mobilité obtenue avec l'invention dans le sens longitudinal.
Les figures 5A à 5C montrent le système de la figure 2 dans trois positions différentes, illustrant la mobilité obtenue avec l'invention dans le sens transversal.

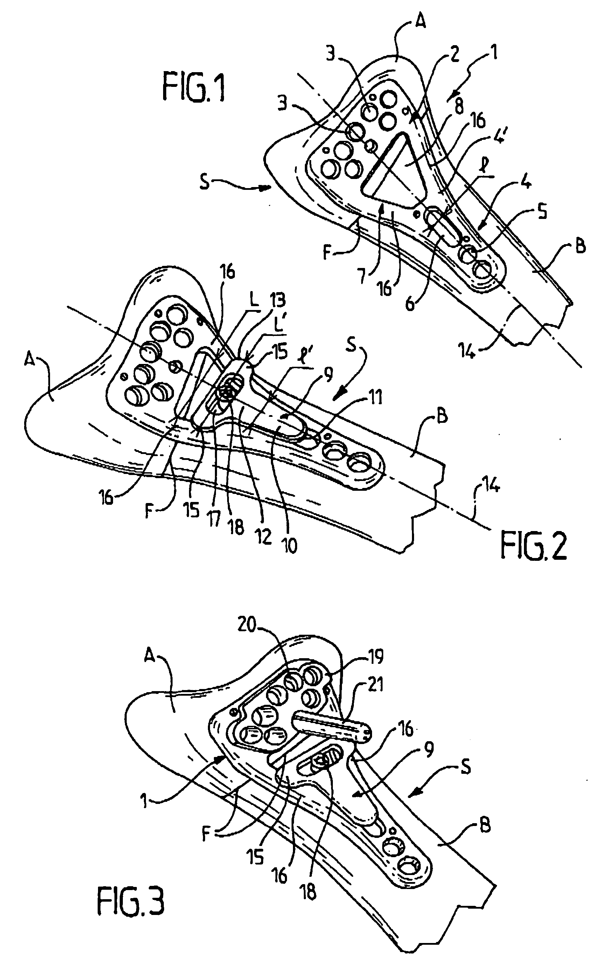

La figure 1 montre un système S comprenant une plaque 1 comportant une partie épiphysaire 2 élargie en forme de palette, pour réduction d'une fracture F entre la partie d'extrémité distale A fracturée d'un radius et le corps de l'os B.

La plaque est munie de premiers trous 3 de fixation pour premières vis d'ancrage (non représentées) par exemple huit trous sur deux rangs, de dimensions déterminées et à des emplacements spécifiques sur l'extrémité de la palette, de façon connue en elle-même.

La plaque 1 comporte également une partie diaphysaire 4 allongée, sensiblement rectangulaire, munie de seconds trous de fixation 5, par exemple deux trous, pour secondes vis d'ancrage non représentées, ici encore de façon connue en elle-même.

La partie diaphysaire 4 comprend de plus un orifice 6 oblong, pour le réglage de la position longitudinale de la plaque sur le radius.

Selon le mode de réalisation de l'invention plus particulièrement décrit ici, la partie épiphysaire 2 comporte une base 7 agrandie de forme sensiblement trapézoïdale, solidaire de l'extrémité proximale 4' de la partie diaphysaire 4, comprenant un évidement 8 sensiblement triangulaire ou trapézoïdal.

En référence à la figure 2, le système S comporte de plus une pièce 9 de réglage mobile comprenant une jambe 10 plus large que le trou oblong de largeur 1. La jambe 10 est munie d'une extrémité 11 plus étroite que la partie 12 de rattachement avec la barre 13 du T, ladite jambe étant donc de largeur 1' > 1 par exemple de 2 mm.

La jambe 10 est munie d'un pion (non visible sur la figure) situé du côté du trou oblong 6, avec lequel le pion coopère à glissement.

Ce pion est par exemple formé par un petit cylindre avec col de guidage, qui vient s'emboîter dans le trou oblong comprenant une marge périphérique sur laquelle le col de guidage repose, le cylindre du pion pénétrant quant à lui dans la partie centrale du trou oblong, dans lequel il peut glisser pour permettre l'ajustage longitudinal.

La pièce 9 présente de plus une barre 13 (la barre du T), de longueur L' dans le sens transversal, perpendiculairement à la direction ou axe longitudinal 14 du trou oblong 6 ou de la jambe 10.

La longueur L' est supérieure à la longueur maximale L de l'évidement 8 dans le sens transversal.

La barre 13 comporte des extrémités 15, qui sont en contact sur une distance suffisante, par exemple quelques millimètres, avec les bords périphériques 16 de l'évidement 8 de la partie épiphysaire élargie.

Selon le mode de réalisation plus particulièrement décrit ici la barre 13 du T comprend une lumière 17, par exemple sensiblement rectangulaire, dont les extrémités sont formées par des portions de cercles.

La longueur de cette lumière dans le sens transversal est sensiblement égale à la longueur maximale L de l'évidement 8, ladite lumière étant propre à la mise en place d'une vis de positionnement 18.

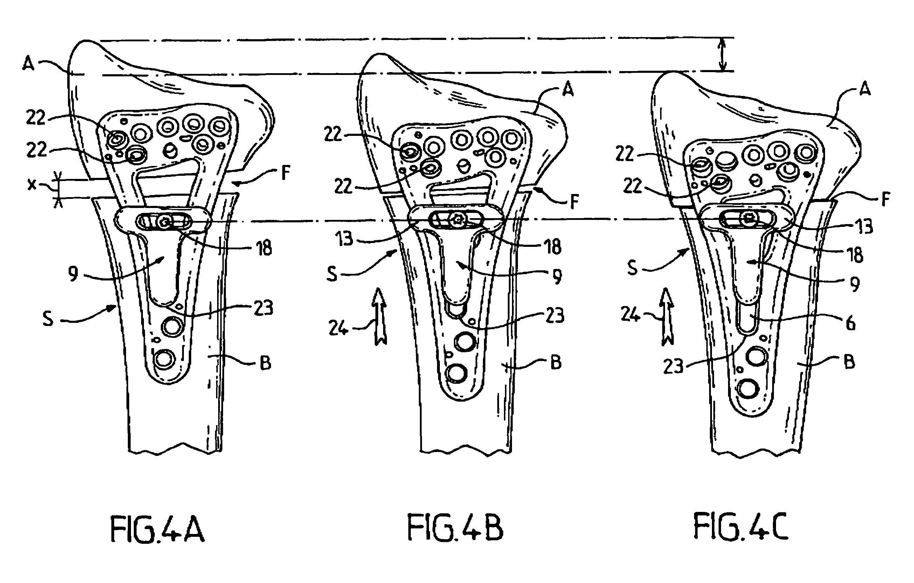

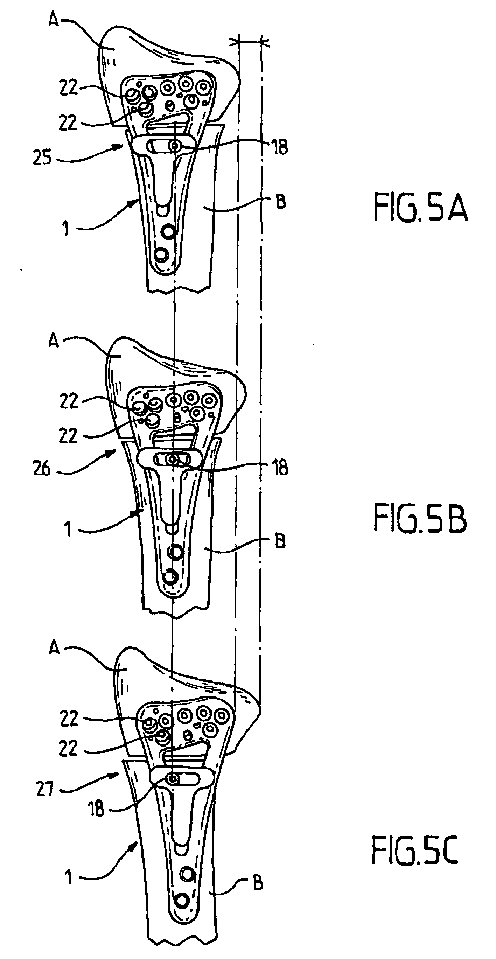

En déplaçant la vis dans la lumière 17 du T et/ou le pion le long du trou oblong 6 cela va permettre de déplacer longitudinalement et/ou transversalement la plaque comme cela sera décrit plus précisément ci-après en référence aux figures 4 et 5.

La figure 3 montre quant à elle, en plus de la plaque 1 et de la pièce 9, un bloc guide 19 de vissage épiphysaire comprenant des orifices 20 cylindriques de guidage, propres à être disposés en vis à vis des trous de fixation 3 pour première vis d'ancrage.

Le système comprend également une tige 21 de positionnement et de maintien du bloc 19 en position, pour percer les trous pour vis d'ancrage.

Les figures 4A à 4C permettent d'illustrer le réglage longitudinal.

Ici l'intérêt de la pièce 9 en T ou glisseur, est de permettre de régler la distance entre les parties A et B avant ou après un premier vissage épiphysaire (vis 22).

Un tel concept autorise donc l'usage d'un système unique, le degré de réglage étant lié à la longueur du trou oblong.

La figure 4A montre une première position dans laquelle le pion (non visible sur la figure) de la jambe du T est situé en butée sur l'extrémité 23 du trou oblong. L'extrémité distale A fracturée du radius est alors, par exemple, fixée par une ou deux vis 22 à la plaque permettant de la solidariser de façon préliminaire.

Dans cette position extrême la partie distale fracturée du radius se trouve donc à une distance x de la partie proximale de l'os.

Au fur et à mesure que l'on déplace le pion à l'intérieur du trou oblong (voir figure 4B) dans le sens de la flèche 24, cela permet de rapprocher la partie fracturée qui voit la distance x se réduire, et ce jusqu'à (voir figure 4C) la mise en contact de la partie fracturée A avec la partie proximale B de l'os comme représentée.

On constate cependant qu'il y a alors un risque de mauvais placement latéral (décalage des côtés), qui va pouvoir être réglé grâce à l'invention, comme cela est maintenant montré en référence aux figures 5A à 5C.

Les figures 5A à 5C montrent successivement une position radiale 25, une position centrée 26 et une position ulnaire 27.

Grâce à la possibilité de faire déplacer la vis 18 à l'intérieur de la lumière 27, on voit en effet que on peut faire bouger latéralement la plaque 1 rendue solidaire (vis 22) de la partie fracturée A, en positionnant la partie diaphysaire allongée de telle façon qu'elle soit centrée (position 26), et ce de façon compréhensible pour l'homme de métier.

On va maintenant décrire ci-après le fonctionnement de la mise en place d'un système d'ostéosynthèse selon l'invention.

On fera notamment ici référence à la figure 3.

Après avoir inséré deux ou trois vis épiphysaires (non représentées) dans les trous 20, on procède à un premier contrôle de l'ensemble S sur l'extrémité du radius par fluroscopie.

Si les positions des vis ne sont pas optimales, on les démonte et on bouge la plaque à l'aide du glisseur.

Grâce à l'invention, et si après fixation des vis épiphysaires 22 on constate que la position épiphyse/diaphyse n'est pas bonne, on peut corriger la position sans démonter la zone épiphysaire.

Pour ce faire, après desserrage de la vis 18 de la pièce 13 ou glisseur on déplace la plaque autour de cette vis sur les deux axes longitudinaux 14, et transversaux (perpendiculaire à l'axe 14) jusqu'au bon positionnement des parties A et B entre elles.

On introduit ensuite les autres vis épiphysaire après avoir donc trouvé la bonne position de la plaque et on serre les deux vis diaphysaires proximales.

Il ne reste plus alors qu'à démonter le glisseur, puis à mettre la dernière vis diaphysaire dans le trou oblong.

On voit que l'intérêt du glisseur est notamment de permettre le réglage de la plaque, avant ou après un premier vissage épiphysaire.

Le degré de réglage est lié à la longueur du trou oblong 6 pour la mobilité longitudinale, et à la longueur de la lumière 17 de la barre du T pour la mobilité latérale.

On aura compris que la pièce en T ou glisseur est uniquement destinée à un usage préopératoire et doit donc être retirée en fin d'opération.

Comme il va de soi et comme il résulte également de ce qui précède, la présente invention n'est pas limitée aux modes de réalisation plus particulièrement décrits. Elle en embrasse au contraire toutes les variantes et notamment celles où la pièce en T est par exemple triangulaire.

## Revendications

1. Système d'ostéosynthèse (S) pour réduction d'une fracture distale (F) d'un os allongé, comprenant une plaque (1) comportant une partie épiphysaire (2) élargie en forme de palette solidaire d'une partie diaphysaire (4) allongée, lesdites parties étant munies de trous de fixation (3, 5) pour vis d'ancrage, ladite partie diaphysaire (4) comprenant un orifice oblong (6) pour réglage de la position longitudinale de la plaque sur l'os,
**caractérisé en ce que** la partie épiphysaire (2) présente une base (7) agrandie comprenant un évidement (8) sensiblement triangulaire ou trapézoïdal,
et **en ce que** le système (S) comporte de plus une pièce (9) de réglage amovible de la plaque sur l'os comprenant une première partie (10) plus large que le trou oblong (6) munie d'un pion agencé pour être inséré dans ledit trou oblong avec lequel il coopère à glissement, et une deuxième partie (13) de longueur L' supérieure à la longueur L de l'évidement et dont les extrémités (15) sont propres à être en contact avec les bords (16) de l'évidement de la partie épiphysaire lorsque le pion est inséré dans le trou oblong, ladite deuxième partie étant munie d'une lumière (17) allongée en vis à vis de l'évidement, ladite lumière (17) étant propre à la mise en place d'une vis (18) de positionnement, de sorte qu'en déplaçant la vis dans la lumière de la pièce de réglage et/ou le pion le long du trou oblong on déplace longitudinalement et/ou transversalement la plaque.

2. Système selon la revendication 1 **caractérisé en ce que** la pièce de réglage amovible est en forme de T, la première partie formant la jambe du T et la deuxième partie la barre du T.

3. Système d'osthéosynthèse selon l'une quelconque des revendications précédentes, **caractérisé en ce que** la partie diaphysaire (4) est sensiblement allongée dans un premier plan et la partie épiphysaire (2) est sensiblement située dans un deuxième plan incliné par rapport au plan de ladite partie diaphysaire.

4. Système selon la revendication 3, **caractérisé en ce que** l'inclinaison entre le plan de la partie epiphysaire et le plan de la partie diaphysaire est compris entre 5° et 15°.

5. Système selon l'une quelconque des revendications précédentes, **caractérisé en ce que** le trou oblong (6) est de longueur comprise entre 6 mm et 12 mm.

6. Système selon l'une quelconque des revendications précédentes, **caractérisé en ce que** la lumière (17) est de longueur comprise entre 12 mm et 14 mm.

7. Système selon l'une quelconque des revendications précédentes, **caractérisé en ce que** l'évidement sensiblement triangulaire présente une surface comprise entre la moitié et les 4/5eme de la base agrandie de la partie épiphysaire.

8. Système selon l'une quelconque des revendications précédentes, **caractérisé en ce que** il comporte de plus un bloc (19) guide de vissage épiphysaire comprenant des orifices (20) de guidage propres à être disposés en vis à vis des trous de fixation des vis d'ancrage de ladite partie épiphysaire.

## Claims

1. Osteosynthesis system (S) for reduction of a distal fracture (F) of an elongated bone, comprising a plate (1) having a widened epiphysary portion (2) in the form of a palette forming one piece with an elongated diaphysary portion (4), said portions being provided with fixing holes (3, 5) for anchoring screws, said diaphysary portion (4) comprising an oblong orifice (6) for adjustment of the longitudinal position of the plate on the bone,
**characterised in that** the epiphysary portion (2) exhibits an enlarged base (7) comprising a substantially triangular or trapezoidal cut-out (8),
and **in that** the system (S) additionally has a detachable adjusting part (9) for adjustment of the plate on the bone comprising a first portion (10) which is wider than the oblong hole (6) and provided with a cylindrical projection arranged to be inserted in said oblong hole with which it cooperates slidingly, and a second portion (13) of length L' greater than the length L of the cut-out and the ends (15) of which are designed to be in contract with the edges (16) of the cut-out of the epiphysary portion when the cylindrical projection is inserted in the oblong hole, said second portion being provided with an elongated opening (17) facing the cut-out, said opening (17) being designed for the placement of a positioning screw (18), such that moving the screw in the opening of the adjusting part and/or the cylindrical projection along the oblong hole moves the plate longitudinally and/or transversely.

2. System according to claim 1, **characterised in that** the detachable adjusting part is T-shaped, the first portion forming the leg of the T and the second portion the crossbar of the T.

3. Osteosynthesis system according to any one of the preceding claims, **characterised in that** the diaphysary portion (4) is substantially elongated in a first plane and the epiphysary portion (2) is substantially located in a second plane which is inclined in relation to the plane of said diaphysary portion.

4. System according to claim 3, **characterised in that** the inclination between the plane of the epiphysary portion and the plane of the diaphysary portion is between 5 degrees and 15 degrees.

5. System according to any one of the preceding claims, **characterised in that** the oblong hole (6) has a length of between 6 mm and 12 mm.

6. System according to any one of the preceding claims, **characterised in that** the opening (17) has a length of between 12 mm and 14 mm,

7. System according to any one of the preceding claims, **characterised in that** the substantially triangular cut-out exhibits a surface area of between half and four fifths of the enlarged base of the epiphysary portion.

8. System according to any one of the preceding claims, **characterised in that** it additionally has an epiphysary screwing guide block (19) comprising guiding orifices (20) designed to be disposed facing the fixing holes of the anchoring screws of said epiphysary portion.

## Patentansprüche

1. Osteosynthesesystem (S) für die Reduktion einer distalen Fraktur (F) eines länglichen Knochens, umfassend eine Platte (1) mit einem schaufelblaltformig verbreiterten Epiphysenbereich (2), welcher mit einem länglichen Diaphysenbereich (4) fest verbunden ist, wobei die genannten Bereiche mit Befestigungslöchern (3, 5) für Verankerungsschrauben versehen sind, wobei der Diaphysenbereich (4) eine längliche Öffnung (6) für die Regelung der Längsposition der Platte auf dem Knochen umfasst,
**dadurch gekennzeichnet,**
**dass** der Epiphysenbereich (2) eine vergrößerte Basis (7) mit einer im Wesentlichen dreieckigen oder trapezförmigen Aussparung (8) aufweist und dass das System (S) ferner ein abnehmbares Teil (9) für die Regelung der Platte auf dem Knochen aufweist mit einem ersten Bereich (10), der breiter als das Langloch (6) ist und mit einem Stift versehen ist, der angeordnet ist, um in das Langloch eingeführt zu werden, mit dem er verschieblich zusammenwirkt, und mit einem zweiten Bereich (13) mit einer die Länge L der Aussparung übersteigenden Länge L', dessen Enden (15) geeignet sind, die Ränder (16) der Aussparung des Epiphysenbereichs zu kontaktieren, wenn der Stift in das Langloch eingeführt wird, wobei der zweite Bereich mit einer länglichen Öffnung (17) gegenüber der Aussparung versehen ist, wobei die genannte Öffnung (17) für das Einsetzen einer Positionierungsschraube (18) geeignet ist, so dass bei Verschiebung der Schraube in der Öffnung des Regelungsteils und/oder des Stifts entlang dem Langloch die Platte in Längs- und/oder Querrichtung verschoben wird.

2. System nach Anspruch 1,
**dadurch gekennzeichnet,**
**dass** das abnehmbare Regelungsteil T-förmig ist, wobei der erste Bereich den Fuß des T und der zweite Bereich den Querstrich des T bilden.

3. Osteosynthesesystem nach einem beliebigen der vorhergehenden Ansprüche,
**dadurch gekennzeichnet,**
**dass** der Diaphysenbereich (4) sich im Wesentlichen in einer ersten Ebene länglich erstreckt und der Epiphysenbereich (2) sich im Wesentlichen in einer zweiten Ebene befindet, die in Bezug auf die Ebene des Diaphysenbereichs geneigt ist.

4. System nach Anspruch 3,
**dadurch gekennzeichnet,**
**dass** die Neigung zwischen der Ebene des Epiphysenbereichs und der Ebene des Diaphysenbereichs zwischen 5° und 15° liegt.

5. System nach einem beliebigen der vorhergehenden Ansprüche,
**dadurch gekennzeichnet,**
**dass** das Langloch (6) eine Länge aufweist, die zwischen 6 mm und 12 mm liegt.

6. System nach einem beliebigen der vorhergehenden Ansprüche,
**dadurch gekennzeichnet,**
**dass** die Öffnung (17) eine Länge aufweist, die zwischen 12 mm und 14 mm liegt.

7. System nach einem beliebigen der vorhergehenden Ansprüche,
**dadurch gekennzeichnet,**
**dass** die im Wesentlichen dreieckige Aussparung eine Fläche aufweist, die zwischen der Hälfte und 4/5 der vergrößerten Basis des Epiphysenbereichs liegt.

8. System nach einem beliebigen der vorhergehenden Ansprüche,
**dadurch gekennzeichnet,**
**dass** es ferner einen Führungsblock (19) für die Epiphysenverschraubung mit Führungsöffnungen (20) aufweist, die geeignet sind, gegenüber des Löchern für die Befestigung der Verankerungsschrauben des Epiphysenbereichs angeordnet zu werden.
